(19) Europäisches Patentamt
European Patent Office
Office européen des brevets

(11) Veröffentlichungsnummer: **0 162 811**
**B1**

(12) # EUROPÄISCHE PATENTSCHRIFT

(45) Veröffentlichungstag der Patentschrift: **11.10.89**

(51) Int. Cl.⁴: **D 06 P 1/642**, C 07 F 1/00

(21) Anmeldenummer: **85810230.4**

(22) Anmeldetag: **15.05.85**

(54) **Verfahren zur fotochemischen Stabilisierung von Polyamidfasermaterial.**

(30) Priorität: **22.05.84 CH 2509/84**

(43) Veröffentlichungstag der Anmeldung: **27.11.85 Patentblatt 85/48**

(45) Bekanntmachung des Hinweises auf die Patenterteilung: **11.10.89 Patentblatt 89/41**

(84) Benannte Vertragsstaaten: **BE CH DE FR GB IT LI NL**

(56) Entgegenhaltungen:
**EP-A- 0 051 188**
**US-A- 3 928 328**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Reinert, Gerhard, Dr.**
**Weiherweg 1/7**
**CH-4123 Allschwil (CH)**
Erfinder: **Back, Gerhard, Dr.**
**Hammerstrasse 5**
**D-7850 Lörrach (DE)**
Erfinder: **Huber-Emden, Helmut, Dr.**
**Sandgrubenstrasse 15**
**CH-4124 Schönenbuch (CH)**

**EP 0 162 811 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur fotochemischen Stabilisierung von ungefärbtem und gefärbtem Polyamidfasermaterial und dessen Mischungen mit anderen Fasern, das gegen fotochemischen Abbau und/oder Ausbleichen der Färbung stabilisierte Fasermaterial als solches, sowie wasserlösliche Kupferkomplexe von Bisazomethinen, Acylhydrazonen, Semicarbazonen und Thiosemicarbazonen aromatischer Aldehyde und Ketone.

Die Verwendung von Kupfersalzen, wie z. B. Kupfersulfat, zur Verbesserung der Lichtechtheit von Färbungen auf Polyamidfasern mit Metallkomplexfarbstoffen ist allgemein bekannt; verwiesen wird auf den Artikel von I. B. HANES in ADR 3 (1980), Seiten 19 und 20. Anorganische oder auch organische Kupfersalze haben jedoch vielfach den Nachteil, dass sie nur ungenügend und ungleichmässig auf die Polyamidfaser aufziehen und daher in hohen Konzentrationen verwendet werden, um den gewünschten Effekt zu erzielen.

Man hat daher versucht, das Kupfer in Form von Verbindungen einzusetzen, die eine Affinität zur Polyamidfaser aufweisen. So wird z. B. in der EP 00 18 775 Kupferphosphat empfohlen, das sich ähnlich wie ein Dispersfarbstoff verhält und entsprechend auf die Nylonfaser aufzieht. Derartige bekannte faseraffine Kupferverbindungen haben jedoch im allgemeinen eine zu geringe Wasserlöslichkeit, was sich ebenfalls negativ auf den Ausziehgrad auswirkt. Darüberhinaus führt das im Färbebad verbleibende Kupfer zu einer starken Abwasserbelastung.

In der EP-A-0 051188 wird ein Verfahren zur Verbesserung der Lichtechtheit von Polyamidfärbungen durch Behandlung mit Kupferkomplexen der Umsetzungsprodukte von Salicylaldehyden mit Aminen beschrieben.

Diese Komplexe besitzen aber eine ausgeprägte Eigenfarbe und eine ungenügende Säurestabilität.

Lösen lassen sich diese Probleme dadurch, dass man anstelle der bekannten, wenig faseraffinen bzw. schlecht wasserlöslichen Kupferverbindungen, sowie der ungeeigneten Kupferkomplexe, organische Kupferkomplexe verwendet, die die angezeigten Nachteile nicht aufweisen.

Gegenstand vorliegender Erfindung ist somit ein Verfahren zur fotochemischen Stabilisierung von ungefärbtem und gefärbtem Polyamid- bzw. Polyurethanfasermaterial, das dadurch gekennzeichnet ist, dass man das Fasermaterial mit einem faseraffinen, wasserlöslichen, organischen sulfogruppenhaltigen Kupferkomplex von Bisazomethinen, Acylhydrazonen, Semicarbazonen oder Thiosemicarbazonen aromatischer Aldehyde oder Ketone behandelt.

Es versteht sich von selbst, dass nur solche Komplexe in Frage kommen, die praktisch farblos oder nur schwach gefärbt sind.

Der Ausdruck fotochemische Stabilisierung bezieht sich im vorliegenden sowohl auf die Lichtechtheit, falls es sich um gefärbtes Material handelt, wie auch auf die Erhaltung der mechanischen Eigenschaften der ungefärbten und gefärbten Polyamid- bzw. Polyurethanfaser. Die Formulierung « gefärbtes Polyamid- bzw. Polyurethanfasermaterial » schliesst optisch aufgehelltes Fasermaterial mit ein.

Besonders gute Ergebnisse werden mit sulfogruppenhaltigen Kupferkomplexen erhalten. Derartige Verbindungen sind gut wasserlöslich und haben zudem eine ausgezeichnete Affinität zur Polyamid- bzw. Polyurethanfaser. Solche Komplexe sind daher bereits in geringen Mengen wirksam. Zudem hat sich gezeigt, dass sie nicht nur die Lichtechtheit des gefärbten Polyamid- bzw. Polyurethanmaterials erhöhen, sondern ganz allgemein die Polyamid- und Polyurethanfaser gegen fotochemischen Abbau schützen und so deren mechanische Eigenschaften, wie Reissfestigkeit und Elastizität weitgehend erhalten.

Unter Bisazomethinen aromatischer Aldehyde und Ketone werden hier Schiff'sche Basen von aliphatischen oder aromatischen Diaminen verstanden, wobei die Aldehyde und Ketone in o-Stellung zum Formyl- bzw. Acylrest eine OH-Gruppe aufweisen. Die Bindung mit dem Metallatom erfolgt über diese beiden OH-Gruppen und die beiden Stickstoffatome im Bisazomethinteil. Es handelt sich demnach hier um vierzähnige Liganden. Die Liganden enthalten eine oder auch mehrere Sulfogruppen, die sich im Aldehyd- bzw. Ketonteil und/oder in der Bisazomethinbrücke befinden.

Bevorzugt zur Anwendung gelangen Bisazomethin-Kupferkomplexe der Formel I

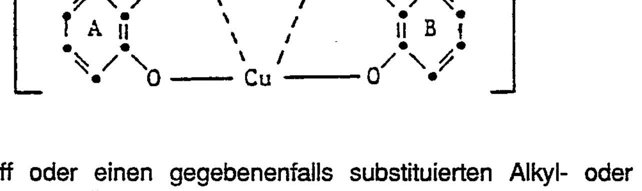

(I)

worin $R_1$ für Wasserstoff oder einen gegebenenfalls substituierten Alkyl- oder Arylrest steht, Y einen gegebenenfalls substituierten Alkylen- oder Cyclohexylenrest bedeutet, n 1, 2 oder 3 ist und die Benzolringe A und B unabhängig voneinander weiter substituiert sein können.

Bezeichnet $R_1$ einen gegebenenfalls substituierten Alkylrest, so kommt vorzugsweise ein $C_1$ bis $C_8$-Alkylrest, insbesondere ein $C_1$ bis $C_4$-Alkylrest in Betracht, der verzweigt oder unverzweigt und gegebenenfalls substituiert sein kann und zwar durch Halogen, wie Fluor, Chlor oder Brom, $C_1$ bis $C_4$-Alkoxy, wie Methoxy oder Aethoxy, durch einen Phenyl- oder Carboxylrest, durch $C_1$ bis $C_4$-Alkoxycarbonyl, wie z. B. den Acetylrest oder durch Hydroxy oder eine mono- oder dialkylierte Aminogruppe. Darüberhinaus kommt auch der Cyclohexylrest in Frage, der ebenfalls substituiert sein kann, wie beispielsweise durch $C_1$ bis $C_4$-Alkyl oder $C_1$ bis $C_4$-Alkoxy.

Bedeutet $R_1$ einen gegebenenfalls substituierten Arylrest, so kommt insbesondere ein Phenyl- oder Naphthylrest in Betracht, der substituiert sein kann durch $C_1$-$C_4$-Alkyl, wie Methyl, Aethyl, Propyl, Isopropyl, Butyl, Isobutyl, sek.-Butyl und tert.-Butyl, $C_1$-$C_4$-Alkoxy, wie Methoxy, Aethoxy, Propoxy, Isopropoxy, Butoxy, Isobutoxy, sek.-Butoxy und ter.-Butoxy, Halogen, wie Fluor, Chlor und Brom, $C_2$-$C_5$-Alkanoylamino, wie Acetylamino, Propionylamino und Butyrylamino, Nitro, Cyano, Sulfo oder eine mono- oder dialkylierte Aminogruppe.

Bedeutet Y einen Alkylenrest, so handelt es sich vor allem um einen $C_2$ bis $C_4$ Alkylenrest, insbesondere eine $-CH_2-CH_2$-Brücke. In Frage kommt aber auch eine durch Sauerstoff oder insbesondere durch Stickstoff unterbrochene $C_2$ bis $C_8$-Alkylenkette und zwar vor allem die $-(CH_2)_3-NH-(CH_2)_3$-Brücke.

Als Substituenten für die Benzolringe A und B kommen in Frage : $C_1$ bis $C_4$ Alkyl, $C_1$ bis $C_4$ Alkoxy, Halogen, wie Fluor, Chlor oder Brom, ferner die Cyano- oder Nitrogruppe.

Die Sulfogruppen, die sich in den Benzoringen A und/oder B befinden, liegen bevorzugt als Alkalimetallsalz, insbesondere als Natriumsalz oder auch als Aminsalz vor.

Insbesondere gelangen im vorliegenden Verfahren die Kupferkomplexe der Formel I zur Anwendung, worin $R_1$ Wasserstoff bedeutet, Y die Aethylenbrücke bezeichnet und n = 2 ist, wobei sich die beiden Sulfogruppen in den Benzolringen A und B befinden und hier vor allem die Komplexe, bei denen die Sulfogruppen jeweils p-ständig zum Sauerstoff angeordnet sind.

Bei den sulfogruppenhaltigen Kupferkomplexen von Acylhydrazonen aromatischer Aldehyde und Ketone handelt es sich in erster Linie um Komplexe der Formel II

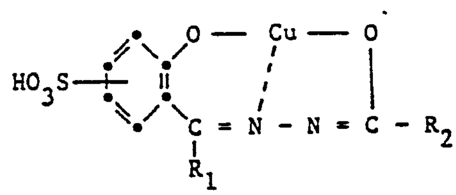

(II)

worin $R_1$ die vorstehend unter Formel I angegebene Bedeutung hat und $R_2$ für Wasserstoff oder einen gegebenenfalls substituierten Alkyl- oder Arylrest steht.

Bedeutet $R_2$ einen Alkylrest, so kann dieser verzweigt oder unverzweigt sein und hat eine Kettenlänge von vorzugsweise 1 bis 8, insbesondere 1 bis 4 C-Atomen. Als Substituenten kommen in Frage Halogen, wie Fluor, Chlor oder Brom, $C_1$ bis $C_4$-Alkoxy, wie Methoxy oder Aethoxy, ferner Phenyl oder Carboxyl, $C_1$ bis $C_4$-Alkoxycarbonyl, wie z. B. Acetyl oder Hydroxy, Mono- oder Dialkylamino.

Bedeutet $R_2$ einen gegebenenfalls substituierten Arylrest, so kommt insbesondere ein Phenyl- oder Naphthylrest in Betracht, der substituiert sein kann durch $C_{1-4}$-Alkyl, wie Methyl, Aethyl, Propyl, Isopropyl, Butyl, Isobutyl, sek.-Butyl und tert.-Butyl, $C_{1-4}$-Alkoxy, wie Methoxy, Aethoxy, Propoxy, Isopropoxy, Butoxy, Isobutoxy, sek.-Butoxy und tert.-Butoxy, Halogen, wie Fluor, Chlor und Brom, $C_{2-5}$-Alkanoylamino, wie Acetylamino, Propionylamino und Butyrylamino, Nitro, Cyano, Sulfo oder eine mono- oder dialkylierte Aminogruppe.

Auch die Komplexe der Formel II werden bevorzugt in neutraler Form, d. h. als Alkalisalz, insbesondere Natriumsalz oder Aminsalz verwendet.

Bevorzugt gelangen solche Komplexe der Formel II zur Anwendung, in denen $R_1$ Wasserstoff und $R_2$ Wasserstoff, Methyl oder insbesondere den Phenylrest bedeutet, vor allem die Komplexe, bei denen sich die Sulfogruppe wiederum in p-Stellung zum Sauerstoff befindet.

Unter Kupferkomplexen von Semicarbazonen bzw. Thiosemicarbazonen werden im vorliegenden in erster Linie Komplexe der Formel III verstanden

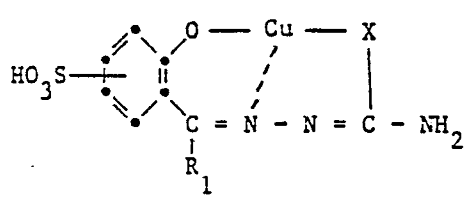

(III)

worin $R_1$ die bereits im Zusammenhang mit der Formel I angegebene Bedeutung hat und X für Sauerstoff oder Schwefel steht. Die Komplexe der Formel III werden ebenfalls bevorzugt als Alkalisalz, insbesondere Natriumsalz bzw. Aminsalz verwendet.

Neben den Kupferkomplexen der Formeln II und III, deren Liganden sich vom Sulfosalicylaldehyd oder den entsprechenden Phenylketonen ableiten, kommen z. B. auch solche in Frage, bei denen anstelle einkerniger, mehrkernige aromatische Aldehyde und Ketone, wie z. B. die 2-Hydroxy-1-naphthaldehydsulfonsäure zum Aufbau des Liganden eingesetzt wurden. Zudem wird darauf hingewiesen, dass die vierte Koordinationsstelle des Metallatoms in den Komplexen der Formeln II und III durch Wasser als Neutralligand besetzt ist.

Die wasserlöslichen faseraffinen Kupferkomplexe werden zweckmässigerweise aus wässrigem Bad appliziert, wobei diese vorteilhaft in einer Menge eingesetzt werden, dass auf 1 g Polyamid- bzw. Polyurethanfasermaterial 5 bis 200 µg, insbesondere 10 bis 100 µg Kupfer kommen, wobei man mit Mengen vom 10 bis 50 µg Metall pro g Polyamid- bzw. Polyurethanmaterial optimale Ergebnisse erreicht.

Werden die Kupferkomplexe zur Stabilisierung von gefärbtem Material verwendet, so kann das Fasermaterial vor, während oder nach der Färbung mit dem Kupferkomplex behandelt werden. Zweckmässigerweise wird der Kupferkomplex direkt dem Färbebad zugesetzt. Die Färbung erfolgt kontinuierlich oder diskontinuierlich.

Unter Polyamidmaterial wird synthetisches Polyamid, wie z. B. Polyamid-6, Polyamid-66 oder auch Polyamid-12, verstanden. Neben den reinen Polyamidfasern kommen vor allem auch Fasermischungen aus Polyurethan und Polyamid in Betracht, so z. B. Trikotmaterial aus Polyamid/Polyurethan im Mischungsverhältnis 70 : 30. Grundsätzlich kann das reine oder gemischte Polyamidmaterial in den verschiedensten Verarbeitungsformen vorliegen, wie z. B. als Faser, Garn, Gewebe oder Gewirk.

Die Färbung erfolgt in üblicher Weise z. B. mit Metallkomplexfarbstoffen oder auch mit Säurefarbstoffen. Als Metallkomplexfarbstoffe werden die bekannten Typen, insbesondere die 1 : 2-Chrom- oder 1 : 2-Kobaltkomplexe von Mono- oder Disazofarbstoffen eingesetzt, die in der Literatur in grosser Zahl beschrieben sind. Neben diesen kommen natürlich auch Farbstoffe aus anderen Farbstoffklassen in Frage, wie z. B. Dispersions- oder auch Küpenfarbstoffe.

Ein weiterer Gegenstand der vorliegenden Erfindung sind die Kupfer-, komplexe der angegebenen Formeln I, II und III worin $R_1$ und $R_2$ Wasserstoff, einen gegebenenfalls substituierten Alkyl oder Arylrest, Y einen gegebenenfalls substituierten Alkylen- oder Cyclohexylenrest und X Sauerstoff oder Schwefel bedeuten, wobei bei den Verbindungen der Formel I mindestens einer der Ringe A und B einen weiteren Substituenten trägt, falls Y Ethylen, Cyclohexylen oder 1,2-Propylen ist, n zwei bedeutet, $R_1$ Wasserstoff ist und die beiden Sulfogruppen jeweils in p-Stellung zum Sauerstoff stehen sowie deren Alkalimetallsalze, wie Kalium- und Lithiumsalze, und insbesondere deren Natriumsalze. Diese werden nach bekannten Methoden erhalten.

Die Kupferkomplexe der Formel I sind beispielsweise auf zwei verschiedenen Wegen zugänglich. So kann man einmal den Aldehyd oder das Keton zunächst metallisieren und anschliessend mit dem entsprechenden Diamin zum fertigen Komplex der Formel I umsetzen. Man kann aber auch zunächst den Liganden aus Aldehyd bzw. Keton und Diamin synthetisieren und anschliessend die Metallisierung durchführen.

Die Einführung der Sulfogruppen kann dabei an verschiedenen Stellen des Syntheseweges erfolgen. Man kann z. B. von einem sulfogruppenhaltigen o-Hydroxyaldehyd bzw. -keton ausgehen und dieses mit einem sulfogruppenfreien oder sulfogruppenhaltigen Diamin zum Bisazomethin umsetzen und anschliessend die Metallisierung durchführen. Es ist auch möglich, einen sulfogruppenhaltigen Aldehyd bzw. Keton zu metallisieren und den Kupferkomplex dann mit einem Diamin zum Bisazomethin-Metallkomplex umzusetzen. Schliesslich kann man auch zunächst den Kupferkomplex des o-Hydroxyaldehyds bzw. -ketons herstellen und diesen dann durch Umsetzung mit einem sulfogruppenhaltigen Diamin, z. B. 3,4-Diaminobenzolsulfonsäure, in den entsprechenden Bisazomethin-Kupferkomplex überführen.

Die Metallisierung wird z. B. mit Kupferhydroxid durchgeführt, beispielsweise indem man zu der wässrigen Lösung des Liganden frisch gefälltes Kupferhydroxid in mindestens äquivalenter Menge zugibt, bis zur Beendigung der Komplexbildung rührt und vom evtl. Kupferhydroxid-Ueberschuss abtrennt.

Vorzugsweise setzt man auf an sich bekannte Art einen sulfogruppenfreien o-Hydroxyaldehyd bzw. ein sulfogruppenfreies o-Hydroxyketon mit einem Diamin zum Bisazomethin um, sulfoniert dieses anschliessend und metallisiert zum Schluss. Die Sulfonierung erfolgt dabei vorzugsweise in 5- bis 50 %igem Oleum, insbesondere in 10- bis 30 %igem Oleum, bei 70 bis 150 °C, vorzugsweise 100 bis 140 °C.

Vorzugsweise werden die vorbeschriebenen drei Schritte, Bildung des Bisazomethins, Sulfonierung und Metallisierung, als Eintopfreaktion, d. h. ohne Isolierung der Zwischenstufen durchgeführt.

Die Acylhydrazone, die Liganden der Komplexe II, werden beispielsweise durch Umsetzen des Aldehyds bzw. Ketons mit dem entsprechenden Monoacylhydrazin und anschliessende Metallisierung gewonnen. Ganz analog lassen sich auch die Komplexe der Formel III herstellen.

Bevorzugt sind insbesondere die Kupferkomplexe der Formeln I und II.

Ganz besonders bevorzugt sind innerhalb der Gruppe I (Komplexe mit Bisazomethinligand) die Kupferkomplexe der Formeln IV

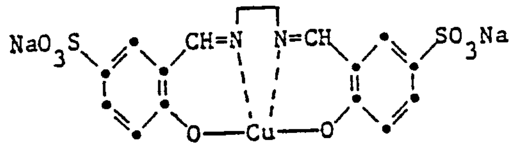

(IV)

und innerhalb der Gruppe II (Komplexe mit Acylhydrazonligand) die Kupferkomplexe der Formeln V, VI und VII

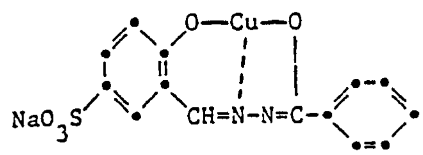

(V)

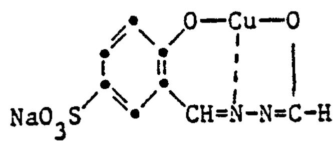

(VI)

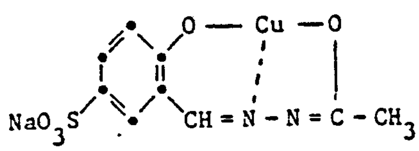

(VII)

Innerhalb der Gruppe III sind die folgenden Verbindungen der Formeln VIII und IX besonders bevorzugt :

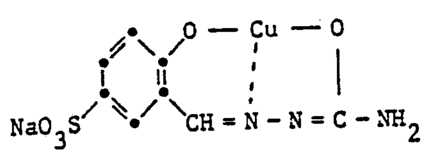

(VIII)

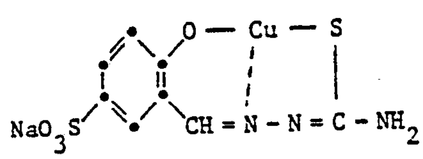

(IX)

Die vierte Koordinationsstelle des Kupfers in den Komplexen der Formeln V, VI und VII ist durch Wasser besetzt, ohne dass dies in den Strukturformeln ausdrücklich vermerkt ist.

Die folgenden Beispiele dienen der Veranschaulichung der Erfindung. Teile bedeuten Gewichtsteile und Prozente Gewichtsprozente. Die Prozentangaben betreffend die Zusätze der einzelnen Behandlungs- bzw. Färbebäder beziehen sich, wenn nicht anders vermerkt, auf das Fasermaterial. Die Temperaturen sind in Grad Celsius angegeben.

Beispiel 1

Stabilisierung von ungefärbtem Polyamidmaterial.

Drei Garnstränge à 10 g aus Polyamid-66 werden in einem Färbeapparat (z. B. einem Färbeapparat mit offenen Behandlungsbädern) mit Flotten (Flottenverhältnis 1 : 20) behandelt, die generell 2 % Ammonsulfat (pH 6,5) enthalten und folgende Zusätze :

Behandlungsbad a) : kein Zusatz.

Behandlungsbad b) : 2,38 mg Kupferkomplex der Formel IV

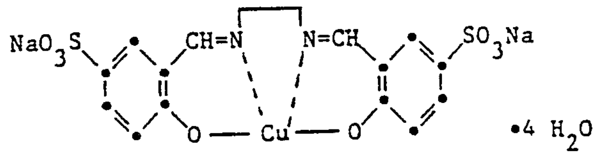

MG = 606,05
Cu = 10,48 %

(IV)

Behandlungsbad c) : 1,6 mg Kupferkomplex der Formel VI

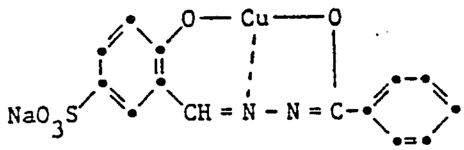

MG = 405,88
Cu = 15,65 %

(VI)

Das Fasermaterial wird in einem Fade-Ometer (Hersteller : Atlas Electric Devices Co., Chicago) während 200 Stunden und einer « black panel temperature » von 83 °C belichtet. Danach wird das Garn nach SNV 197.461 (Richtlinien der Schweizerischen Normenvereinigung) auf seine Reissfestigkeits- und Dehnungswerte geprüft. Es werden folgende Ergebnisse erhalten :

| Behandlungsbad | Cu-Gehalt * µg/g | Cu-Gehalt * % | Reissfestigkeit (%) | Dehnung (%) |
|---|---|---|---|---|
| a) | - | - | 23 | 32 |
| b) | 23 | 0,0023 | 76,2 | 81,2 |
| c) | 25 | 0,0025 | 81,6 | 84,9 |
| Blindbehandlung ohne Belichtung | - | - | 100 | 100 |

* laut Mikroanalyse des Polyamid-Fasermaterials.

## Beispiel 2

Verbesserung der Lichtechtheit einer Beige-Färbung.

Wie üblich vorgewaschenes Polyamid-6-Trikotmaterial wird wie folgt behandelt :

a) Herkömmlich gefärbt

Das Färbebad wird mit 2 % Ammonsulfat beschickt (pH 6,5) und das Polyamid-Material ca. 5 Minuten bei 40 °C vorlaufen gelassen. Danach erfolgt die Zugabe folgender Mengen in Wasser gelöster Farbstoffe (jeweils auf das Fasermaterial berechnet) :

0,04% Farbstoff 1

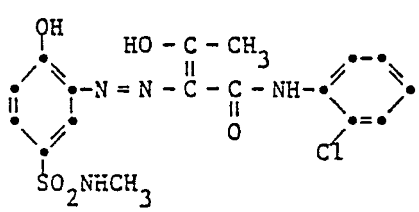

1:2-Co-Komplex (gelb)

(Fortsetzung)

0,025 %
Farbstoff 2

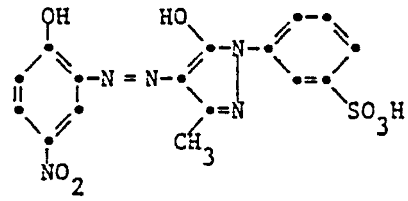

+

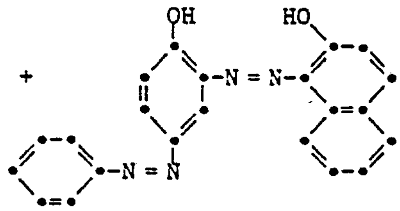

1:2-Cr-Komplex (braun)

0,003%
Farbstoff 3

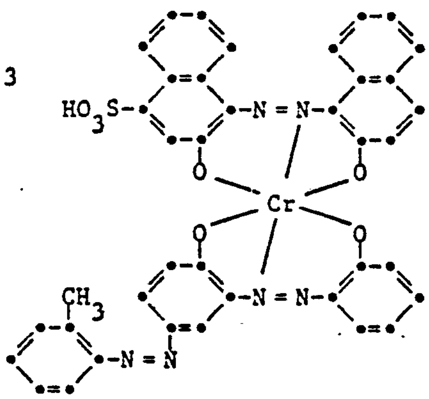

1:2-Cr-Komplex (schwarz)

Anschliessend wird das Färbebad mit einer Geschwindigkeit von 1 1/2 °C pro Minute auf 95 °C erhitzt und 45 Minuten bei dieser Temperatur gehalten. Nach einer Färbezeit von 10 Minuten wird der pH-Wert der Färbeflotte durch Zusatz von 80 %iger Essigsäure auf 5,2 eingestellt. Ist die Färbezeit abgelaufen, lässt man das Bad auf 70 °C abkühlen, entnimmt das gefärbte Material, spült warm, zentrifugiert und trocknet bei 100 °C.

b) Unter Zusatz von faseraffinen Cu-Komplexen

Es wird wie unter 2a) vermerkt verfahren, jedoch zu Färbebeginn 0,023 8 % Komplex der Formel IV bzw. in einer weiteren Färbung 0,016 % Komplex der Formel V zugesetzt. Die Mengen an Cu-Komplex der Formel IV bzw. V entsprechen einer theoretischen Cu-Menge von 25 µg/g Polyamid.

Das Trikotmaterial gefärbt nach a) und b) wird der Lichtechtheitsbewertung nach SN-ISO 105-BO2 (Xenon) und einem Heissbelichtungstest nach DIN 75.202 (prov.) (Fakra), unterworfen.

Ergebnisse :

| Färbung | Lichtechtheitsnoten | |
|---|---|---|
| | SNV 195.809 | Xenon Heissbelichtung |
| ohne Zusatz eines Cu-Komplexes | 5 | 4 |
| mit Cu-Komplex IV | 6 | 6 - 7 |
| mit Cu-Komplex V | 5 - 6 | 6 |

## Beispiel 3

Verbesserung der Lichtechtheit einer Helloliv-Färbung.

Die Färbung erfolgt wie im Beispiel 2 beschrieben mit dem Unterschied, dass folgende Farbstoffkombination zum Färben dient und die Mengen der Zusätze von Cu-Komplex IV und V verdoppelt werden (entspricht einer theoretischen Cu-Menge von 50 µg/g Polyamid).

0,05%

Farbstoff 4

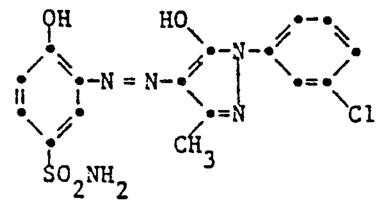

1:2-Co-Komplex (gelb)

0,25%

Farbstoff 5

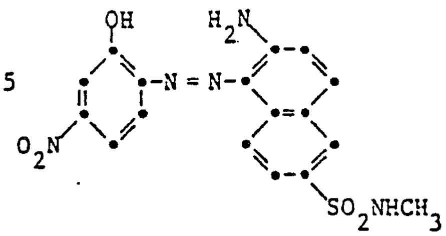

+

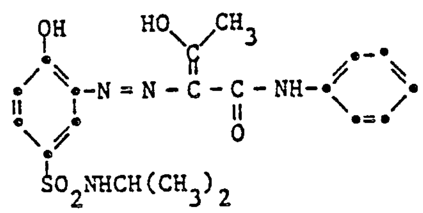

1:2-Co-Komplex (grün)

0,035%

Farbstoff 3 gemäss Beispiel 2 (schwarz)

Die Testung der Färbungen wurde wie im Beispiel 2 vermerkt vorgenommen.

Ergebnisse :

| Färbung | Lichtechtheitsnoten | |
|---|---|---|
| | SNV 195.809 | Xenon Heissbelichtung |
| ohne Zusatz eines Cu-Komplexes | 5 | 4 |
| mit Cu-Komplex IV | 6+ | -6 |
| mit Cu-Komplex V | 5 - 6 | 6 - 7 |

## Beispiel 4

Fotochemische Stabilisierung von gefärbtem und ungefärbtem Polyamidmaterial.

Sechs Garnstränge à 100 g aus Polyamid-66 werden mit und ohne Zusatz von 2,5 mg Cu-Komplex IV blindbehandelt sowie gefärbt mit den in Beispiel 2 und 3 angegebenen Farbstoffkombinationen. In allen Fällen erfolgt die Behandlung wie im Beispiel 2 beschrieben.

Das Garn wird sodann in einem Xenotest-Gerät (siehe Beispiel 2) nach SN-ISO 105-BO2 während 1 000 Stunden belichtet und danach nach SNV 197.461 auf seine Reissfestigkeit und Dehnung geprüft. Es wurden folgende Ergebnisse erhalten :

| Garnmuster | Reissfestigkeit (%) | Dehnung (%) |
|---|---|---|
| Blindbehandlung unbelichtet | 100 | 100 |
| Blindbehandlung belichtet | | |
| ohne Cu-Komplex | 36 | 60,7 |
| mit Cu-Komplex IV | 90,6 | 96,2 |
| Beigefärbung belichtet | | |
| ohne Cu-Komplex | 74 | 82,7 |
| mit Cu-Komplex IV | 87,5 | 88 |
| Olivefärbung belichtet | | |
| ohne Cu-Komplex | 77 | 84,7 |
| mit Cu-Komplex IV | 85,3 | 89,8 |

Obwohl die bei der Färbung verwendeten Farbstoffe zu einer Klasse von Metallkomplexfarbstoffen gehören, die das Polyamidmaterial vor fotochemischem Abbau schützen, kann durch Zusatz des Cu-Komplexes der Formel IV eine deutliche Verbesserung der Reissfestigkeit des Fasermaterials erreicht werden.

Beispiel 5

Herstellung des Cu-Komplexes der Formel IV.

a) Herstellung des Liganden

0,45 g Aethylendiamin, gelöst in 2 ml Wasser, werden zu einer siedenden Lösung von 4,0 g Salicylaldehydsulfonsäure-Na-Salz in 75 ml 90 %igem Isopropanol zugerührt. Es fällt sofort ein voluminöser Niederschlag aus. Man lässt noch 15 Minuten am Rückfluss kochen, filtriert in der Hitze und wäscht mit heissem 90 %igem Isopropanol nach. Man erhält 3,35 g einer hellgelblichen Festsubstanz (94 % d. Th.).

Analyse : $C_{16}H_{14}N_2Na_2O_8S_2$
Berechnet : C 40,7 H 3,0 N 5,9
Gefunden : C 40,8 H 3,2 N 5,9
Die Substanz lässt sich aus den selben Edukten auch in wässriger Lösung gewinnen.

b) Metallisierung

Eine wässrige Lösung von 2,5 g Kupfer-II-sulfat wird in 25 ml 1-normale Natronlauge eingerührt. Man saugt das ausgefallene Kupferhydroxid ab und wäscht gut nach. Der Filterrückstand wird mit 30 ml Wasser verrührt, während man portionsweise 1 g des Produktes gemäss Beispiel 5a) zugibt. Man rührt noch 30 Minuten, saugt vom nicht umgesetzten Kupferhydroxid ab und versetzt das Filtrat mit 250 ml Aethanol. Der Niederschlag wird abgesaugt, mit 90 %igem Aethanol gewaschen und im Exsiccator getrocknet. Man erhält 0,8 g Kupferkomplex der Formel IV als hellviolettes Pulver (64 % d. Th.). Das Produkt enthält 4 Mol Kristallwasser.

Analyse : $C_{16}H_{20}CuN_2Na_2O_{12}S_2$
Berechnet : C 31,7 H 3,3 N 4,6 Cu 10,5
Gefunden : C 32,0 H 3,5 N 5,0 Cu 10,7
Aus wässriger Lösung lässt sich die Substanz mit Kochsalz aussalzen oder durch Versetzen mit Kupfer-(II)-Ionen als schwerlösliches Kupfersalz ausfällen. Die Verbindung entsteht auch aus dem

literaturbekannten Kupferkomplex des Salicylaldehydsulfonsäure-Na-Salzes [M. Calvin, N. C. Melchior JACS 70, 3270-3 (1948)] durch Umsetzen mit Aethylendiamin in siedendem verdünntem Alkohol, allerdings in geringerer Reinheit.

## Beispiel 6

Herstellung des Cu-Komplexes der Formel V.

2,24 Teile des Natriumsalzes von 2-Hydroxybenzaldehyd-5-sulfonsäure werden in 20 Teilen Wasser bei 70 °C gelöst. Nach Zugabe von 1,36 Teilen Benzoesäurehydrazid wird das Reaktionsgemisch 1 Stunde bei 90 bis 95 °C gehalten, wobei eine klare, gelbe Lösung der Hydrazonverbindung von folgender Konstitution entsteht :

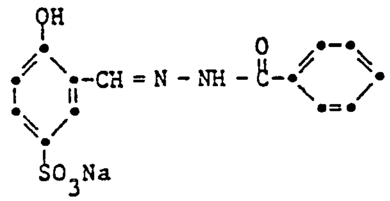

Zur Ueberführung in den Kupferkomplex wird die erhaltene Reaktionslösung mit einer Lösung von 1,7 Teilen $CuCl_2 \cdot 2\,H_2O$ und 4 Teilen kristallisiertem Natriumacetat in 10 Teilen Wasser versetzt und anschliessend 30 Minuten bei 70 bis 75 °C gehalten. Der als grüngelber Niederschlag ausgefallene 1 : 1-Kupferkomplex wird durch Zugabe von 2n-Natriumhydroxidlösung bis zum pH-Wert 9,0 bis 9,5 vollständig gelöst. Die gelbstichig grüne Lösung wird zur Trockne eingeengt, wobei 7 Teile Kupferkomplex in Form eines olivgrünen, leicht wasserlöslichen Pulvers erhalten werden.

Zur Herstellung des Kupferkomplexes kann anstelle von Kupferchlorid auch ein beliebiges anderes Kupfersalz oder frisch gefälltes Kupferhydroxid verwendet werden.

Verwendet man zur Herstellung der Hydrazonverbindung anstelle von Benzoesäurehydrazid 0,60 Teile Ameisensäurehydrazid oder 0,74 Teile Essigsäurehydrazid, so entstehen beim Metallisieren nach obenstehendem Verfahren analoge 1 : 1-Kupferkomplexe, die auf Polyamidfasermaterial appliziert ähnlich stabilisierende Eigenschaften zeigen.

## Beispiel 7

Fotostabilisierung von Polyamidmaterial gefärbt mit verscheidenen Metallkomplexfarbstoffen.

Sechs Garnstränge aus Polyamid-66 von je 10 g werden in einem Laborfärbeapparat bei einem Flottenverhältnis von 1 : 20 mit Flotten behandelt, die neben 2 % Ammonsulfat (pH 6,8) und 0,5 g/l 4-Nonylphenol · 10 Ethylenoxid als Dispergator, die in der Tabelle vermerkten Zusätze enthalten. Man behandelt zunächst 10 Minuten bei 40 °C und erhitzt das Bad dann während 30 Minuten auf 95 °C. Nach 10 Minuten setzt man 2 % Essigsäure (80 %ig) zu (pH 5,2) und auf 95 °C. Nach 10 Minuten setzt man 2 % Essigsäure (80 %ig) zu (pH 5,2) und färbt weitere 35 Minuten bei 95 °C, kühlt sodann auf 70 °C ab, spült das Garn warm und kalt mit Wasser und trocknet in einem Umluftofen bei 100 °C.

In einem Fade-ometer wird das Garn während 200 Stunden bei 83 °C belichtet (siehe Beispiel 1) und danach nach SNV 197.461 die Reissfestigkeit ermittelt.

Ergebnisse :

| Behandlung | Zusätze | Reissfestigkeit (%) |
|---|---|---|
| 1 | keine | 33,1 |
| 2 | 0,05% Cu-Komplex IV | 82,2 |
| 3 | 0,075% Farbstoff 6 | 22,3 |
| 4 | 0,075% Farbstoff 6<br>0,05% Cu-Komplex IV | 60,7 |
| 5 | 0,075% Farbstoff 7 | 48,4 |
| 6 | 0,075% Farbstoff 7<br>0,050% Cu-Komplex IV | 82,8 |
| Ausgangsmaterial, unbelichtet | | 100 |

Der Farbstoff 6 mit der Formel

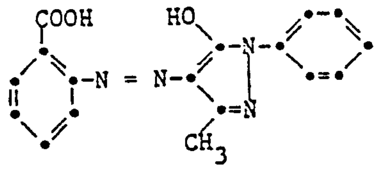

1:2-Cr-Komplex (gelb)

gehört zu der Gruppe von Farbstoffen, die einen negativen Einfluss auf die Lichtbeständigkeit der Polyamidfaser haben.

Der Farbstoff 7 mit der Formel

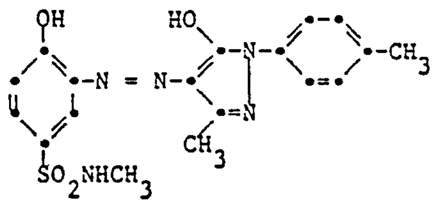

1:2-Co-Komplex (gelb)

gehört hingegen zu einer Gruppe von Farbstoffen, die das Polyamidmaterial gegen fotochemischen Abbau schützen.

Aus der Tabelle des Beispiels 8 geht eindeutig hervor, dass der Kupferkomplex IV, auch bei Verwendung von Farbstoffen, die einen fotochemischen Abbau der Polyamidfaser begünstigen, einen rapiden Abfall der Reissfestigkeit aufzuhalten vermag.

## Beispiel 8

Stabilisierung von Polyamidmaterial gefärbt mit Dispers- bzw. Säurefarbstoffen.

Sechs Garnstränge aus Polyamid-66 werden wie in Beispiel 8 beschrieben behandelt, belichtet und geprüft, jedoch unter Verwendung der folgenden Farbstoffe

Farbstoff 8

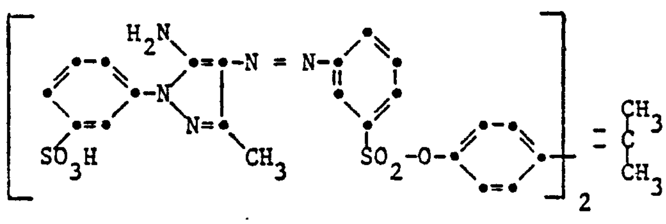

Farbstoff 9

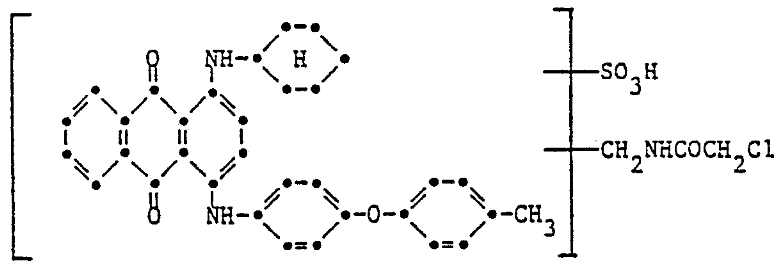

Farbstoff 10

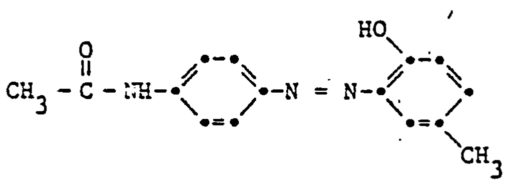

Zusätze zum Färbebad und Ergebnisse

| Behandlung | Zusätze | Reissfestigkeit % | Dehnung % |
|---|---|---|---|
| 1 | 0,075% Farbstoff 8 | 39,6 | 68,7 |
| 2 | 0,075% Farbstoff 8<br>0,050% Cu-Komplex IV | 72,4 | 97,4 |
| 3 | 0,075% Farbstoff 9 | 41,3 | 71,2 |
| 4 | 0,075% Farbstoff 9<br>0,050% Cu-Komplex IV | 82,0 | 103,5 |
| 5 | 0,075% Farbstoff 10 | 28,0 | 48,8 |
| 6 | 0,075% Farbstoff 10<br>0,050% Cu-Komplex IV | 87,6 | 112,5 |

Die Ergebnisse zeigen, dass auch mit Nicht-Metallkomplexfarbstoffen gefärbtes Polyamidmaterial durch Zusatz eines faseraffinen, wasserlöslichen Kupferkomplexes gegen fotochemischen Abbau geschützt wird.

Beispiel 9

Fotochemische Stabilisierung von Polyamid/Polyurethan-Trikot.

Zwei Trikotstücke von je 10 g aus Polyamid-66/Lycra® 70 : 30, vorgereinigt, werden in einem Färbeapparat bei einem Flottenverhältnis von 1 : 20 mit und ohne 0,075 % Cu-Komplex IV wie in Beispiel 8 beschrieben behandelt.

Anschliessend wird das Trikotmaterial in einem Fade-ometer 80 und 160 Stunden bei 83 °C (siehe Beispiel 1) belichtet und nach SNV 197.461 auf seine Reiss- und Dehnungswerte geprüft.

Ergebnisse

| Behandlung | | Verlust an (%) Reissfestigkeit | Dehnung |
|---|---|---|---|
| blind | unbelichtet | - | - |
| | 80 Stunden belichtet | 79,9 | 38,3 |
| | 160 Stunden belichtet | 94,4 | 52,5 |
| mit 0,075% | 80 Stunden belichtet | 27 | 1 |
| Cu-Komplex IV | 160 Stunden belichtet | 50,4 | 13,3 |

Durch Anwendung eines wasserlöslichen, faseraffinen Kupferkomplexes wird auch bei Polyamid/Polyurethan-Mischgewebe der fotochemische Abbau stark verzögert.

Beispiel 10

Fotochemische Stabilisierung von gefärbtem Polyamid/Polyurethan-Trikot.

Vier Trikotstücke aus Polyamid 6,6/Polyurethan 70/30 werden wie in Beispiel 8 beschrieben mit nachfolgend vermerkten Farbstoffen mit und ohne Cu-Komplex IV gefärbt :

Färbungen 1 und 2 mit 0,1 % Farbstoff 10 (siehe Seite 20),

Färbungen 3 und 4 mit 0,1 % Farbstoff 11.

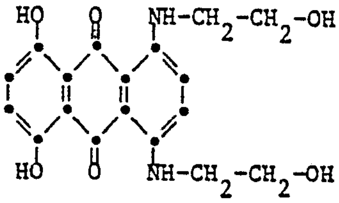

(11)

Die Färbungen 2 und 4 enthalten zusätzlich noch 0,05 % Kupferkomplex der Formel IV.

Die Testung der Färbungen erfolgt durch Bestimmung der Lichtechtheiten (SN-ISO 105-BO2 und DIN 75.202 (prov.), sowie von Reissfestigkeit und Dehnung (nach SNV 198.461) nach der 150 stündigen Belichtung im Fade-ometer bei 83°.

Ergebnisse

| Behandlung | Zusätze | Lichtechtheiten | | nach Belichtung 150 h/83° | |
|---|---|---|---|---|---|
| | | Xenon | Fakra | Reissfestigkeit [%] | Dehnung [%] |
| 1 | 0,1% Farbstoff 10 | 6 | 5 | 6,7 | 42,1 |
| 2 | 0,1% Farbstoff 10 + 0,05% Cu-Komplex IV | 6-7 | 5-6 | 54,8 | 79,8 |
| 3 | 0,1% Farbstoff 11 | 4-5 | 4-5 | 7,1 | 47,1 |
| 4 | 0,1% Farbstoff 11 + 0,05% Cu-Komplex IV | 6 | 5-6 | 41,7 | 72,7 |
| 0 | unbehandelt, unbelichtet | | | 100 | 100 |

Der Zusatz des Cu-Komplexes IV bewirkt eine gute fotochemische Stabilisierung des Fasermaterials und eine Verbesserung der Lichtechtheiten.

Beispiel 11

Herstellung des Kupferkomplexes der Formel IV.

268 g Bis-salicyliden-ethylendiamin werden innerhalb 2 Minuten unter Rühren in 747 g 21,4 %iges Oleum eingetragen, wobei die Substanz exotherm in Lösung geht (ca. 130°). Man erhitzt noch 1 1/2 Stunden auf ca. 105° und rührt bis die Reaktionsmasse dick wird. Nach Abkühlen auf 50° giesst man 3 Liter Wasser zu und stellt mit 30 %iger Natronlauge langsam auf pH 7,5, wobei infolge Exothermie eine Innentemperatur von 73-75° erreicht wird und alles in Lösung geht. Man gibt eine 60° warme Lösung von 200 g Kupferacetat-monohydrat in 1,5 Liter Wasser zu, stellt mit 30 %iger Natronlauge erneut auf pH 7,5 und lässt auf Raumtemperatur abkühlen. Der Niederschlag wird durch Druckfiltration isoliert, mit 5 %iger Natriumsulfatlösung gewaschen und bei 70° im Vakuum getrocknet und an der Luft 15 Stunden konditioniert (wobei er Kristallwasser aufnimmt). Man erhält 534,5 g der violettblauen Verbindung IV, die nach Analyse 5,25 % Natriumsulfat enthält (Reinausbeute 86 %) ; für die meisten Verwendungszwecke ist das Produkt in diesem Zustand rein genug.

Zur Entfernung des beigemengten Natriumsulfates verrührt man eine 10 g-Probe des Rohprodukts mit 60 ml in Natronlauge, saugt ab, wäscht dann mit Ethanol neutral ; nach Trocknen an Luft erhält man 9,0 g violettblaue Kristalle der Formel IV, enthaltend 3 Mol Kristallwasser.

$C_{16}H_{18}CuN_2Na_2O_{11}S_2$
Berechnet : C 32,7 H 3,1 Cu 10,8 N 4,7
Gefunden : C 32,4 H 3,1 Cu 10,6 N 4,9.

Beispiel 12

Kupferkomplex des N,N'-Bis[1-(2-hydroxy-5-sulfophenyl)-ethyliden]-ethylendiamins (Di-Natriumsalz).

14,8 g N,N'-Bis-[1-(2-hydroxyphenyl)-ethyliden]-ethylendiamin werden innerhalb 1 Minute in 37 g 21,6 %iges Oleum eingetragen, wobei es sich exotherm löst (ca. 150°). Man hält noch 1 Stunde bei ca.

105°, kühlt auf Raumtemperatur ab, giesst 150 ml Wasser zu und stellt mit 30 %iger Natronlauge langsam auf pH 10,0 und rührt 5 Stunden bei 60°. Nach Abkühlen saugt man ab, wäscht den Rückstand mit 2n Natronlauge sulfatfrei und dann mit Ethanol neutral. Der Filterrückstand wird in 270 ml Wasser gelöst, von ungelösten Verunreinigungen abgesaugt und das Filtrat mit 900 ml Ethanol versetzt ; das ausgefallene Produkt wird abgesaugt, im Exsiccator getrocknet und an Luft über Nacht konditioniert (wobei es Kristallwasser aufnimmt). Man erhält 7,1 g hellviolettrosafarbene Kristalle, die 3 Mol Kristallwasser enthalten.

$C_{18}H_{22}CuN_2Na_2O_{11}S_2$
Berechnet : C 35,1 H 3,6 Cu 10,3 N 4,5
Gefunden : C 35,4 H 3,4 Cu 10,2 N 4,5.

Beispiele 13 bis 25

Arbeitet man analog wie im Beispiel 11 beschrieben, verwendet jedoch anstelle von Bissalicylidenethylendiamin die Bisazomethine aus den in der folgenden Tabelle verzeichneten Aldehyden und Diaminen, so erhält man die entsprechenden Kupferkomplexe. Die Sulfogruppen sind dabei jeweils in p-Stellung zu den OH-Gruppen angeordnet.

| Beispiel | Aldehyd | Diamin |
|---|---|---|
| 13 | 2-Hydroxy-3-chlor-benzaldehyd | Ethylendiamin |
| 14 | 2-Hydroxy-3-methoxybenzaldehyd | do |
| 15 | 2-Hydroxybenzaldehyd | 1,2-Diaminopropan |
| 16 | do | 1-Phenylethylendiamin |
| 17 | do | cis-1,2-Diaminocyclohexan |
| 18 | do | trans-1,2-Diaminocyclohexan |
| 19 | do | 1,3-Diaminopropan |
| 20 | do | 2,2-Dimethyl-1,3-diaminopropan |
| 21 | do | 2-Hydroxy-1,3-diaminopropan |
| 22 | do | Bis-(3-aminopropyl)-amin |

Beispiel 23

N,N'-Bis-(2-hydroxy-5-sulfo-benzyliden)-ethylendiamin-di-Na-Salz.

0,9 g Ethylendiamin lässt man langsam zu einer siedenden Lösung von 8 g Sulfosalicylaldehyd-Na-Salz in 200 ml 90 %igem Isopropanol laufen. Man kocht noch 15 Minuten am Rückfluss, filtriert heiss ab, wäscht mit 90 %igem Isopropanol heiss nach und erhält als Rückstand nach dem Trocknen 6,85 g einer hellgelben Festsubstanz.

$C_{16}H_{14}N_2Na_2S_2$
Berechnet : C 40,7 H 3,0 N 5,9
Gefunden : C 40,5 H 3,3 N 5,9.

Beispiel 24

Cu-Komplex des N,N'-Bis-[1-(2-hydroxy-5-sulfo-phenyl)-ethyliden]-ethylendiamin-di-Na-Salzes.

Zu einer Lösung von N,N'-Bis-[1-(2-hydroxy-5-sulfo-phenyl)-ethyliden]-ethylendiamin-di-Na-Salz in 10 ml Wasser gibt man 6 mMol feuchtes Kupferhydroxyd, das aus wässriger Kupfersulfatlösung mit

überschüssiger Natronlauge frisch gefällt, abfiltriert und neutralgewaschen wurde. Man rührt 3 Stunden, saugt ab und giesst das Filtrat in 50 ml Ethanol. Der Niederschlag wird abgesaugt, mit Ethanol gewaschen und getrocknet. Man erhält den entsprechenden Kupferkomplex in Form hellviolettrosafarbener Kristalle.

Beispiel 25

N,N'-Bis-(2-hydroxy-3-sulfo-5-chlor-benzyliden)-ethylendiamin-di-Na-Salz.

3,37 g N,N'-Bis-(2-hydroxy-5-chlor-benzyliden)-ethylendiamin werden in 9,6 g 25 %igem Oleum 1 Stunde bei 100° gerührt. Man kühlt ab, giesst 30 ml Eiswasser zu, gibt so viel 30 %ige Natronlauge zu, bis der pH-Wert mehrere Stunden konstant 7,5 bleibt, saugt vom ausgefallenen Produkt ab, wäscht mit Wasser sulfatfrei und trocknet. Zwecks Reinigung verrührt man mit 100 ml Chloroform, saugt ab und trocknet. Man erhält 3,3 g einer gelben Festsubstanz, enthaltend 3 Mol Kristallwasser.

$C_{16}H_{18}Cl_2N_2Na_2O_{11}S_2$

| | | | | | |
|---|---|---|---|---|---|
| Berechnet: | C 32,3 | H 3,0 | Cl 11,9 | N 4,7 | S 10,8 |
| Gefunden: | C 32,3 | H 2,9 | Cl 11,7 | N 4,8 | S 10,8. |

Beispiel 26

Arbeitet man wie im Beispiel 25 beschrieben, verwendet jedoch als Diamin N,N'-Bis-(2-hydroxy-5-brom-benzyliden)-ethylendiamin, so erhält man N,N'-Bis-(2-hydroxy-3-sulfo-5-brom-benzyliden)-ethylendiamin-di-Na-Salz.

Beispiel 27

Cu-Komplex des N,N'-Bis-(2-hydroxy-3-sulfo-5-brom-benzyliden)-ethylendiamin-di-Na-Salzes.

1,35 g Kupferacetat-monohydrat werden in 20 ml Dimethylformamid durch leichtes Erwärmen gelöst. Man trägt 4,52 g der feingepulverten gemäss Beispiel 26 erhaltenen Verbindung ein und rührt eine Stunde. Dann versetzt man mit 40 ml Wasser, wobei sich zunächst alles löst, dann aber das Produkt rasch ausfällt. Nach dem Absaugen wäscht man mit Wasser und Ethanol und lässt an Luft trocknen. Man erhält 4,48 g einer ziegelroten Festsubstanz, enthaltend 2 Mol Kristallwasser.

$C_{16}H_{14}Br_2CuN_2Na_2O_{10}S_2$

| | | | | | | |
|---|---|---|---|---|---|---|
| Berechnet: | C 26,4 | H 1,9 | Br 22,0 | Cu 8,7 | N 3,9 | S 8,8 |
| Gefunden: | C 26,6 | H 2,1 | Br 21,8 | Cu 9,1 | N 4,1 | S 8,7. |

Beispiel 28

Arbeitet man wie im Beispiel 27 beschrieben, verwendet jedoch als Ausgangssubstanz die gemäss Beispiel 25 erhaltene Verbindung, so erhält man den Kupferkomplex von N,N'-Bis-(2-hydroxy-3-sulfo-5-chlor-benzyliden)-ethylendiamin-di-Na-Salz in Form einer hellvioletten Festsubstanz.

Beispiel 29

Kupferkomplex des N,N'-Bis-[2-hydroxy-6-sulfo-naphthyl-(1)-methylen]-ethylendiamin-di-Na-Salz.

3,72 g N,N'-Bis-[2-hydroxy-naphthyl-(1)-methylen]-ethylendiamin werden mit 10 g 100 %iger Schwefelsäure 1 Stunde bei 100° gerührt. Nach Abkühlen giesst man auf 30 g Eis, stellt mit 30 %iger Natronlauge auf pH 7,5, rührt 1 Stunde. saugt ab, wäscht mit Eiswasser sulfatfrei und trocknet. Man erhält 5,25 g einer gelben Festsubstanz, enthaltend 1 Mol Kristallwasser.

$C_{24}H_{20}N_2Na_2O_9S_2$

| | | | | |
|---|---|---|---|---|
| Berechnet: | C 48,8 | H 3,4 | N 4,7 | S 10,9 |
| Gefunden: | C 49,0 | H 3,4 | N 4,9 | S 10,8. |

1,67 g Kupferacetat-monohydrat werden in 40 ml Dimethylformamid unter leichtem Erwärmen gelöst. Man gibt 4,95 g der feingepulverten vorstehend beschriebenen gelben Festsubstanz und 4 ml Wasser zu und erhitzt 2 Stunden auf 100°. Nach Abkühlen wird abgesaugt, mit Dimethylformamid und Ethanol nachgewaschen und an Luft getrocknet. Man erhält 5,05 g einer olivgelben Festsubstanz.

$C_{24}H_{16}CuN_2Na_2O_8S_2$

| | | | | | |
|---|---|---|---|---|---|
| Berechnet: | C 45,5 | H 2,5 | Cu 10,0 | N 4,4 | S 10,1 |
| Gefunden: | C 45,4 | H 2,8 | Cu 10,0 | N 4,5 | S 10,0. |

Beispiel 30

Eine Lösung einer 0,76 g 3,4-Diaminobenzoesäure äquivalenten Menge Bis-(3-aminopropyl)amin in 5 ml Natronlauge gibt man bei 70° zu einer Suspension von 2,35 g Cu-Komplex des Sulfosalicylaldehyd-Na-Salzes in 30 ml Ethanol. Man rührt 1 Stunde bei 70° unter Stickstoff, kühlt ab und saugt ab. Der Filterrückstand wird in 20 ml Wasser gelöst, mit einigen Tropfen in Natronlauge stark alkalisch gestellt und von einer ausgefallenen Verunreinigung abgesaugt. Das Filtrat davon giesst man in 100 ml Ethanol, filtriert den Niederschlag ab, wäscht mit Ethanol neutral und trocknet an Luft. Man erhält einen hellgrünen Kupferkomplex enthaltend 5 Mol Kristallwasser.

Beispiel 31

2,24 Teile 2-Hydroxybenzaldehyd-5-sulfonsäure in Form des Natriumsalzes werden in 50 Teilen Wasser von 70° unter Rühren gelöst. Nach Zugabe von 1,11 Teilen Semicarbacid-Hydrochlorid wird das Reaktionsgemisch durch Zutropfen verdünnter Natriumhydroxidlösung auf bleibend pH 7,0 gestellt und noch 1 Stunde bei 70 bis 75° gerührt. Es entsteht eine klare, schwach grünlichgelbe Lösung der folgenden Hydrazonverbindung :

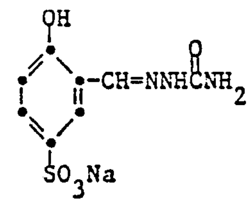

Zur Ueberführung in den entsprechenden Kupferkomplex wird die Reaktionslösung mit einer Lösung von 1,70 Teilen Kupfer-II-chlorid-dihydrat und 4,0 Teilen kristallisiertem Natriumacetat in 30 Teilen Wasser versetzt und anschliessend 30 Minuten bei 70 bis 75° gehalten. Der als hell blaugrüner Niederschlag ausgefallene 1 : 1-Kupferkomplex wird durch Filtration isoliert und mit verdünnter Natriumchloridlösung gewaschen. Er löst sich in einer Mischung aus Wasser und Dimethylformamid mit blaugrünem Farbton.

Beispiel 32

2,24 Teile 2-Hydroxybenzaldehyd-5-sulfonsäure in Form des Natriumsalzes werden unter den im vorhergehenden Beispiel angegebenen Bedingungen mit 0,91 Teilen Thiosemicarbacid zur Hydrazonverbindung von folgender Konstitution umgesetzt :

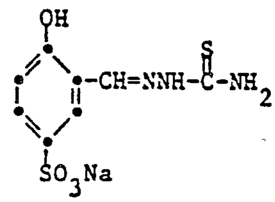

Der entsprechende 1 : 1-Kupferkomplex ist analog dem oben erwähnten Komplex zugänglich und löst sich in Wasser mit olivbraunem Farbton.

**Patentansprüche**

1. Verfahren zur fotochemischen Stabilisierung von ungefärbtem und gefärbtem Polyamid- bzw. Polyurethanfasermaterial, dadurch gekennzeichnet, dass man das Fasermaterial mit einem faseraffinen, wasserlöslichen organischen sulfogruppenhaltigen Kupferkomplex von Bisazomethinen, Acylhydrazonen, Semicarbazonen oder Thiosemicarbozonen aromatischer Aldehyde oder Ketone behandelt.

2. Verfahren gemäss Anspruch 1, dadurch gekennzeichnet, dass man einen Kupferkomplex der Formel I verwendet

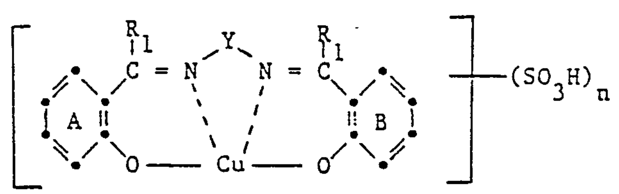

(I)

worin $R_1$ für Wasserstoff oder einen gegebenenfalls substituierten Alkyl- oder Alkylrest steht, Y einen gegebenenfalls substituierten Alkylen- oder Cyclohexylrenrest bedeutet, n 1, 2 oder 3 ist und die Benzolringe A und B unabhängig voneinander weiter substituiert sein können.

3. Verfahren gemäss Anspruch 1, dadurch gekennzeichnet, dass man einen Kupferkomplex der Formel II verwendet

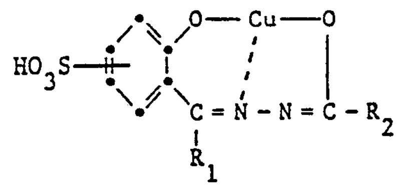

(II)

worin $R_1$ die unter Formel I angegebene Bedeutung hat und $R_2$ für Wasserstoff oder einen gegebenenfalls substituierten Alkyl- oder Arylrest steht.

4. Verfahren gemäss Anspruch 1, dadurch gekennzeichnet, dass man einen Kupferkomplex der Formel III verwendet

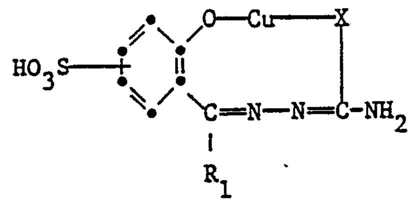

(III)

worin $R_1$ die unter Formel I angegebene Bedeutung hat und X für Sauerstoff oder Schwefel steht.

5. Verfahren gemäss Anspruch 1, dadurch gekennzeichnet, dass man den Kupferkomplex direkt dem Färbebad zusetzt.

6. Verfahren gemäss Anspruch 1, dadurch gekennzeichnet, dass man den Kupferkomplex in einer Menge einsetzt, dass auf 1 g Polyamid- bzw. Polyurethanmaterial 5 bis 200 µg Metall kommen.

7. Verfahren gemäss Anspruch 1, dadurch gekennzeichnet, dass man den Kupferkomplex in einer Menge einsetzt, dass auf 1 g Polyamid- bzw. Polyurethanmaterial 10 bis 100 µg Metall kommen.

8. Kupferkomplexe der Formeln I, II und III

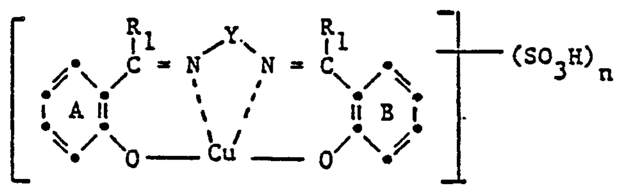

(I)

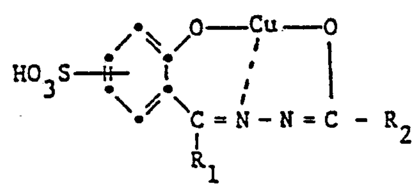

(II)

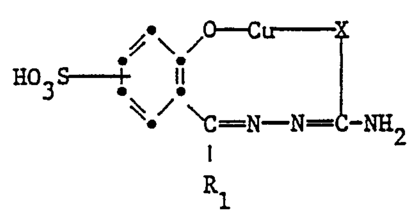

(III)

und deren Alkalimetallsalze,
worin $R_1$ und $R_2$ Wasserstoff, einen gegebenenfalls substituierten Alkyl- oder Arylrest, Y einen gegebenenfalls substituierten Alkylen- oder Cyclohexylenrest und X Sauerstoff oder Schwefel bedeuten, wobei bei den Verbindungen der Formel I mindestens einer der Ringe A und B einen weiteren Substituenten trägt, falls Y Ethylen, Cyclohexylen oder 1,2-Propylen ist, n zwei bedeutet, $R_1$ Wasserstoff ist und die beiden Sulfogruppen jeweils in p-Stellung zum Sauerstoff stehen.

9. Kupferkomplex gemäss Anspruch 8, der Formel II, dadurch gekennzeichnet, dass $R_1$ Wasserstoff und $R_2$ Wasserstoff, Methyl oder Phenyl bedeuten und die Sulfogruppe in p-Stellung zum Sauerstoff ist.

10. Kupferkomplex gemäss Anspruch 8, der Formel III, dadurch gekennzeichnet, dass $R_1$ Wasserstoff ist und die Sulfogruppe in p-Stellung zum Sauerstoff angeordnet ist.

## Claims

1. A process for the photochemical stabilization of undyed and dyed polyamide or polyurethane fibre material, characterized in that the fibre material is treated with a water soluble organic copper complex of a bisazomethine, acylhydrazone, semicarbazone or thiosemicarbazone of an aromatic aldehyde or ketone, said copper complex containing sulfo groups and having an affinity for the fibres.

2. A process according to claim 1, characterized in that a copper complex of formula I is used

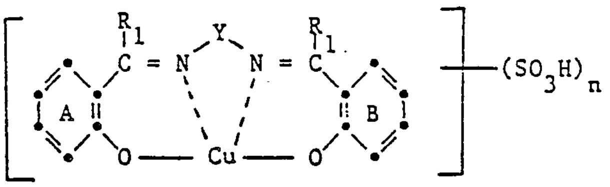
(I)

wherein $R_1$ is hydrogen or an optionally substituted alkyl or aryl radical, Y is an optionally substituted alkylene or cyclohexylene radical, n is 1, 2 or 3 and the benzene rings A and B may be further substituted independently of one another.

3. A process according to claim 1, characterized in that a copper complex of formula II is used

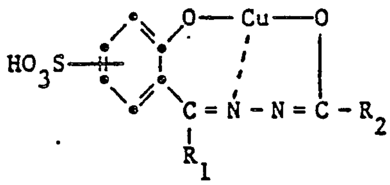
(II)

wherein $R_1$ is as defined under formula I and $R_2$ is hydrogen or an optionally substituted alkyl or aryl radical.

4. A process according to claim 1, characterized in that a copper complex of formula III is used

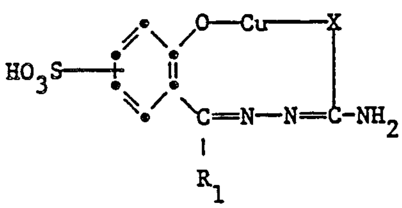
(III)

wherein $R_1$ is as defined under formula I and X is oxygen or sulfur.

5. A process according to claim 1, characterized in that the copper complex is added directly to the dye bath.

6. A process according to claim 1, characterized in that the copper complex is used at such a quantity that 5-200 µg of metal are applied on each 1 g of polyamide or polyurethane material.

7. A process according to claim 1, characterized in that the copper complex is used at such a quantity that 10-100 µg of metal are applied on each 1 g of polyamide or polyurethane material.

8. Copper complexes of formulae I, II and III

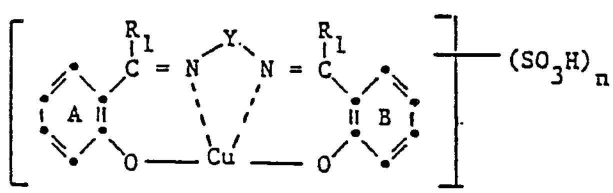
(I)

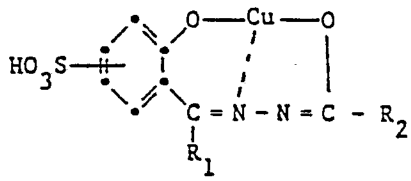

(II)

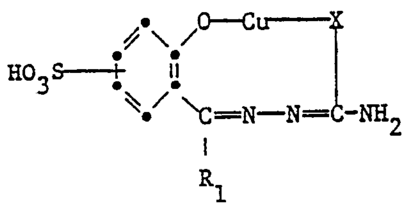

(III)

and their alkali metal salts, wherein $R_1$ and $R_2$ are hydrogen, an optionally substituted alkyl or aryl radical, Y is an optionally substituted alkylene or cyclohexylene radical, X is oxygen or sulfur, at least one of the rings A and B in the compounds of formula I carrying a further substitute, in the case that Y is ethylene, cyclohexylene, or 1,2-propylene, n is two, $R_1$ is hydrogen, and the two sulfo groups are in each instance in the p-position to the oxygen.

9. Copper complexes according to claim 8, of formula II, characterized in that $R_1$ is hydrogen, $R_2$ is hydrogen, methyl or phenyl and the sulfo group is in the p-position to the oxygen.

10. Copper complexes according to claim 8, of formula III, characterized in that $R_1$ is hydrogen and the sulfo group is in the p-position to the oxygen.

**Revendications**

1. Procédé de stabilisation photochimique d'un matériau en fibres de polyamide ou de polyuréthanne, teint ou non, caractérisé en ce que le matériau en fibres est traité par un complexe organique de cuivre et de bisazométhine, d'acylhydrazone, de semicarbazone ou de thiosemicarbazone d'aldéhyde ou de cétone aromatique, porteur de groupements sulfo, soluble dans l'eau et fibrophile.

2. Procédé d'après la revendication 1, caractérisé en ce que l'on utilise un complexe de cuivre de formule I :

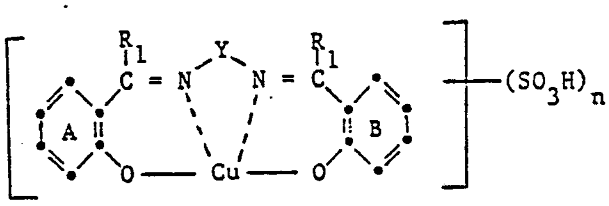

(I)

où $R_1$ représente un hydrogène ou reste alkyle ou aryle éventuellement substitué, Y un reste alkylène ou cyclohexylène éventuellement substitué, n vaut 1, 2 ou 3 et les cycles benzéniques A et B peuvent encore être substitués indépendamment l'un de l'autre.

3. Procédé d'après la revendication 1, caractérisé en ce que l'on utilise un complexe de cuivre de formule II :

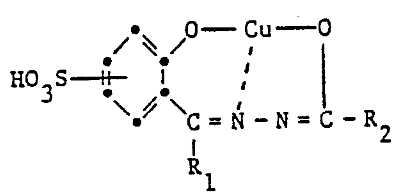

(II)

où $R_1$ a la même signification que dans la formule I et $R_2$ représente un hydrogène ou un reste alkyle ou aryle éventuellement substitué.

4. Procédé d'après la revendication 1, caractérisé en ce que l'on utilise un complexe de cuivre de formule III :

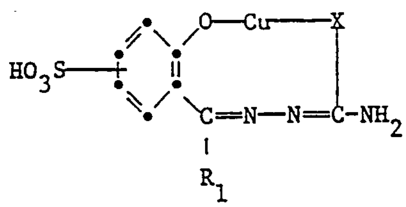

(III)

où $R_1$ a la même signification que dans la formule I et X représente un oxygène ou un soufre.

5. Procédé d'après la revendication 1, caractérisé en ce que l'on ajoute directement le complexe de cuivre au bain de teinture.

6. Procédé d'après la revendication 1, caractérisé en ce que l'on utilise le complexe de cuivre en une quantité telle que 5 à 200 μg de métal se fixent sur 1 g de matériau de polyamide ou de polyuréthanne.

7. Procédé d'après la revendication 1, caractérisé en ce que l'on utilise le complexe de cuivre en une quantité telle que 10 à 100 μg de métal se fixent sur 1 g de matériau de polyamide ou de polyuréthanne.

8. Complexes de cuivre de formules I, II et III :

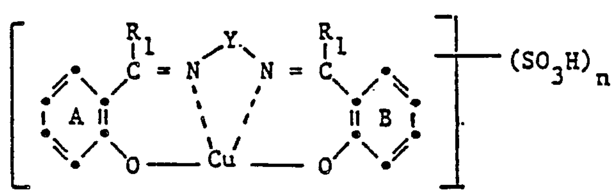

(I)

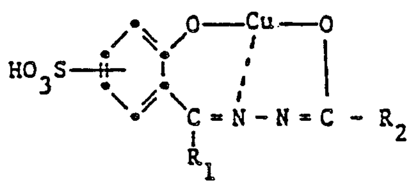

(II)

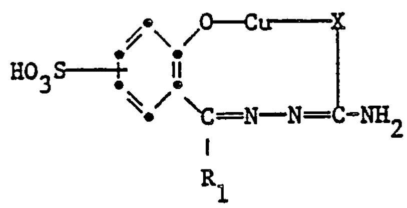

(III)

et leurs sels de métaux alcalins, où $R_1$ et $R_2$ représentent un hydrogène ou un reste alkyle ou aryle éventuellement substitué, Y un reste alkylène ou cyclohexylène éventuellement substitué et X un oxygène ou un soufre, et où, en ce qui concerne les composés de formule I, un au moins des cycles A et B porte un autre substituant, dans le cas où Y est un éthylène, un cyclohexylène ou un propylène-1,2, n vaut 2, $R_1$ est un hydrogène et les deux groupements sulfo sont chacun en position para par rapport à l'oxygène.

9. Complexe de cuivre d'après la revendication 8, de formule II, caractérisé en ce que $R_1$ représente un hydrogène, $R_2$ un hydrogène, un méthyle ou un phényle et le groupement sulfo est en position para par rapport à l'oxygène.

10. Complexe de cuivre d'après la revendication 8, de formule III, caractérisé en ce que $R_1$ représente un hydrogène et le groupement sulfo se trouve en position para par rapport à l'oxygène.